# EUROPEAN PATENT SPECIFICATION

(11) **EP 4 368 989 B1**
(45) Date of publication and mention of the grant of the patent: **18.06.2025**
(21) Application number: 23197886.7
(22) Date of filing: 18.09.2023
(51) Int. Cl.: G01N 33/00, A01G 7/00

(54) **AGRICULTURAL MONITORING SYSTEM**
LANDWIRTSCHAFTLICHES ÜBERWACHUNGSSYSTEM
SYSTÈME DE SURVEILLANCE AGRICOLE

(30) Priority: 28.09.2022 JP 2022154757
(43) Date of publication of application: 15.05.2024
(73) Proprietor: Sintokogio, Ltd., Nagoya-shi, Aichi 450-6424 (JP)
(72) Inventor: Toda, Takahiro, Nagoya-shi, Aichi, 450-6424 (JP)
(74) Representative: Hoffmann Eitle

(56) References cited:
- WO-A1-2019/237200
- JP-A- 2010 032 252
- JP-A- 2021 193 894
- JP-A- 2022 002 591

## Description

### TECHNICAL FIELD

The present disclosure relates to monitoring system.

### BACKGROUND

Japanese Patent Application Publication No. 2020-46386 discloses an environment measuring device that measures a growth environment of a plant. The apparatus has a gas sensor disposed adjacent to the plant. The gas sensor measures the carbon dioxide concentration around the plant.

WO2019237200A1 relates to an agriculture system and related methods. In one aspect, there is a growing system comprising a plurality of sensors for sensing one or both of parameters of a plant or parameters of an environment in which the plant is being grown; an environmental control system for controlling one or more growing conditions of the plant; a controller coupled to the plurality of sensors and configured to: receive sensor data from the plurality of sensors; determine whether parameters based at least in part on the sensor data match one or more performance criteria; and cause the environmental control system to perform an adjustment to at least one growing condition of the plant in response to a determination that the parameters do not match the one or more performance criteria.

JP2021193894A relates to a soil diagnostic system comprising an odor sensor 21 which measures a kind and a volume of gas sent out from soil on which plants grow while scanning the entire soil by scanning means and a distribution creation part 33 which creates a distribution of gas generated in the soil being an object to be measured from a result measured by the odor sensor 21. Further, the system also comprises an estimation processing part 36 which estimates a presence of replant failure occurrence or a presence of soil bacteria if necessary.

JP2010032252A relates to an evaluating device of the carbon dioxide consumption function of plants under illumination comprising an illumination/sensor section that is arranged opposed to a plant to be measured and that is equipped with an illuminating body and a carbon dioxide concentration sensor on its surface, a displaying device that is connected with the carbon dioxide concentration sensor via an electric wiring extending from the illumination/sensor section and that displays data based on the carbon dioxide concentration value detected by the sensor, and an electric power supply that is electrically connected with each of the illuminating body, the carbon dioxide concentration sensor, and the displaying device.

JP2022002591A relates to a display device including a display shelf for displaying a plurality of plants, an irradiation part for irradiating the displayed plants with light, and a control part for controlling the irradiation part. The control part highlights the irradiated plant by controlling at least one of control parameters, which comprises irradiation intensity, a blinking rate, an irradiation wavelength and an irradiation range, for at least one of the plurality of plants.

### SUMMARY

The gas sensor described in Japanese Patent Application Publication No. 2020-46386 may detect not only gas generated from the plant but also a component caused by disturbance such as gas generated from soil.

The present invention provides a monitoring system that includes a first gas sensor, a second gas sensor, and a controller. The first gas sensor is configured to be disposed in a first agricultural land where a plant is cultivated, and to detect a gas component. The second gas sensor is configured to be disposed in second agricultural land where the plant is not cultivated, and has the same gas detection function as the first gas sensor. The controller is configured to output information about gas generated from the plant based on the first detection result of the first gas sensor and the second detection result of the second gas sensor, this information being obtained by excluding a common gas component from the first detection result as the information about gas generated from the plant when the common gas component is included between the first detection result and the second detection result.

Accordingly, gas generated from a plant can be accurately measured.

### BRIEF DESCRIPTION OF THE DRAWINGS

FIG. 1 is a block diagram of a monitoring system according to an embodiment.
FIG. 2 is a diagram schematically illustrating a monitoring system.
FIG. 3 is a flowchart showing the operation of the monitoring system.
FIG. 4 is a flowchart showing details of the gas information calculation process.

### DETAILED DESCRIPTION

Hereinafter, embodiments of the present disclosure will be described in detail with reference to the drawings. In the description of the drawings, the same elements are denoted by the same reference numerals, and redundant description is omitted. The dimensional ratios in the drawings are not necessarily consistent with those in the description.

### [Example of Monitoring System]

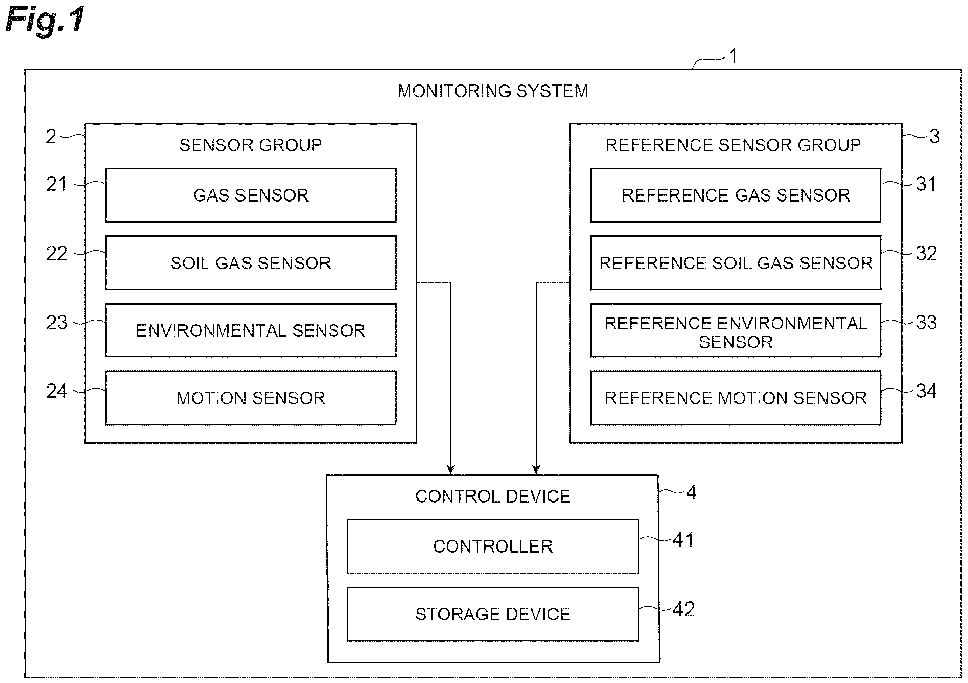

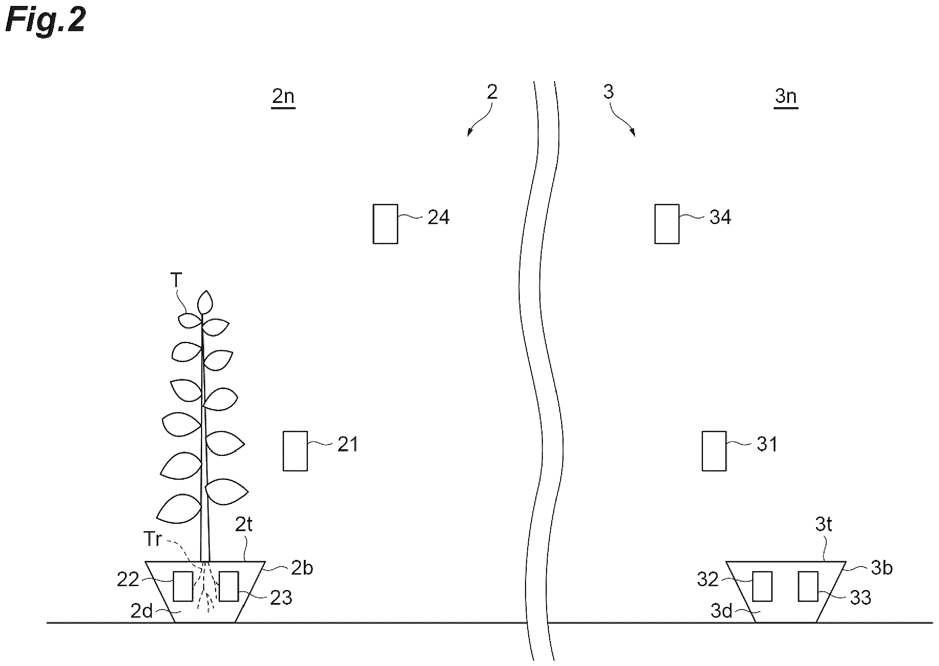

FIG. 1 is a block diagram of a monitoring system 1 in accordance with an embodiment. FIG. 2 is a diagram schematically showing the monitoring system 1. The monitoring system 1 shown in FIGS. 1 and 2 is a system for monitoring the state of a plant T. The plant T is a plant to be measured, and examples thereof include agricultural crops such as tomatoes and fruits such as apples. Examples of states of the plant T include health state of the plant T and growth state of the plant T.

The monitoring system 1 includes a sensor group 2, a reference sensor group 3, and a control device 4. The sensor group 2 is a sensor group for measuring the state of the plant T. The sensor group 2 includes at least one gas sensor. The gas sensor detects a gas component. The reference sensor group 3 is a sensor group for measuring a state of a comparative environment with respect to an environment in which the plant T is cultivated. The reference sensor group 3 is provided with a reference gas sensor corresponding to the gas sensor contained in the sensor group 2. The sensor group 2 and the reference sensor group 3 are configured to communicate with the control device 4 via wireless or wired communication. Each of the sensor group 2 and the reference sensor group 3 is configured to transmit a detection data or the like to the control device 4.

The control device 4 outputs information about the plant T based on the detection result of the gas sensor included in the sensor group 2 and the detection result of the gas sensor included in the reference sensor group 3. The information about the plant T is information about gas generated from the plant T. The information about the plant T may further be information about a state of the plant T estimated based on a gas component generated from the plant T. The sensor group 2, the reference sensor group 3, and the control device 4 will be described in detail below.

As shown in FIG. 2, the sensor group 2 is disposed in the first agricultural land 2n where the plant T is cultivated. The agricultural land is land and place intended for the cultivation of plants and the work of plowing field. Examples of agricultural land include culture medium, paddy fields, upland fields, tree land, and pasture land. The first agricultural land 2n is a land and a place where the plant T is cultivated in an agricultural land. In the example shown in FIG. 2, the first agricultural land 2n includes a culture medium 2b and the plant T is cultivated in the culture medium 2b. The culture medium 2b has a soil 2d. The reference sensor group 3 is disposed in the second agricultural land 3n where the plant T is not cultivated. Second agricultural land 3n is agricultural land where the plant T is not cultivated. As an example, the second agricultural land 3n is located in the same farm, plastic house or the like as the first agricultural land 2n. In the example shown in FIG. 2, the second agricultural land 3n includes a culture medium 3b that is in the same environment as the culture medium 2b of the first agricultural land 2n. The culture medium 3b contains a soil 3d of the same quality as the soil 2d. That is, the environment of the second agricultural land 3n is the same as the environment of the first agricultural land 2n except that the plant T is not cultivated.

Examples of the sensor group 2 include a gas sensor 21 (the first gas sensor), a soil gas sensor 22 (an example of a third gas sensor), an environmental sensor 23 (an example of a first environment sensor), and a motion sensor 24 (an example of a first motion sensor). The gas sensor 21 is provided above a ground surface 2t of the first agricultural land 2n and detects the gas component. By ground surfaces of agricultural lands is meant the surfaces of the soil 2d on which the plant T are cultivated. In the example shown in FIG. 2, the ground surface 2t of the first agricultural land 2n is the surface of soil 2d in the culture medium 2b, and the gas sensor 21 is provided above the culture medium 2b. The gas sensor 21 is provided adjacent to the plant T by a string (not shown) or the like so as to detect gas (odor substance) generated from, for example, leaves, stems, fruits, or the like of the plant T.

The substance (the gas component) detected by the gas sensor 21 includes, for example, at least one of green leaf volatiles, terpenes, sulfur-based substances, and moldy odor substances. The gas sensor 21 may also detect other substances in addition to the green leaf volatiles, the terpenes, the sulfur-based substances, and the moldy odor substances.

The green leaf volatiles are volatile substances such as aldehydes, alcohols, and esters thereof having a skeleton of six carbon atoms. In detail, the green leaf volatiles include hexenol (leaf alcohol), cexenal (leaf aldehyde), or the like. The terpenes include organic compounds having an unsaturated hydrocarbon of (C₅H₈)ₙ as a basic skeleton and terpene alcohols. Specifically, the terpenes include α-pinene, β-caryophyllene or the like. The sulfur-based substances include a sulfide compound such as hydrogen sulfide that emits a putrid odor of plant T. Specific examples of the sulfur-based substances include dimethyl sulfide or the like. Examples of the moldy odor substances include mold, trichloroanisole, geomine, methylisoborneol or the like.

An example of the gas sensor 21 is electrochemical formulas sensor. The electrochemical formulas sensor may be a VOC (Volatile Organic Compounds) sensor. For example, the VOC sensor including polyaniline as an organic film and tin oxide (SnO₂) as a metal semiconductor may be used. The gas sensor 21 is not limited to the VOC sensor, and may be any sensor capable of detecting gas. The gas sensor 21 may be configured with multiple sensors when detecting multiple different odor gases.

The soil gas sensor 22 is disposed below the ground surface 2t of the first agricultural land 2n and detects the gas component. In the example shown in FIG. 2, the soil gas sensor 22 is provided in the culture medium 2b. The soil gas sensor 22 is provided in the vicinity of a root Tr of the plant T to detect gas emanating from the root Tr of the plant T. The soil gas sensor 22 may be a sensor capable of detecting gas, and is VOC sensor, for example.

The environmental sensor 23 is disposed in the soil 2d of first agricultural land 2n. The environmental sensor 23 detects at least one of moisture and electrical conductivity of the soil 2d of the first agricultural land 2n. The environmental sensor 23 includes a moisture sensor when the environmental sensor 23 detects moisture in the soil 2d. The environmental sensor 23 includes an electrical conductivity (EC) sensor when the environmental sensor 23 detects the electrical conductivity of the soil 2d. The environmental sensor 23 may further detect values other than the moisture and electrical conductivity of the soil 2d. The position of the environmental sensor 23 is not particularly limited as long as the growth environment of the plant T can be detected.

The motion sensor 24 is disposed in the first agricultural land 2n. The motion sensor 24 detects people present in the first agricultural land 2n. The motion sensor 24 may be, for example, an infrared sensor disposed in the first agricultural land 2n. In addition, the motion sensor 24 may be constituted by an imaging device disposed in the first agricultural land 2n and a computer that analyzes an image captured by the imaging device, for example. The motion sensor 24 is not particularly limited as long as it is configured to be able to detect a person present in first agricultural land 2n. The motion sensor 24 is fixed in the vicinity of the plant T by a string (not shown), for example.

Each detection data (an example of a detection result) of the gas sensor 21, the soil gas sensor 22, the environmental sensor 23, and the motion sensor 24 is output to the control device 4.

Examples of the reference sensor group 3 include a reference gas sensor 31 (the second gas sensor), a reference soil gas sensor 32 (an example of a fourth gas sensor), a reference environment sensor 33 (an example of a second environment sensor), and a reference motion sensor 34 (an example of a second motion sensor). The reference gas sensor 31 is provided above a ground surface 3t of the second agricultural land 3n. In the example shown in FIG. 2, the ground surface 3t of the second agricultural land 3n is the surface of soil 3d in the culture medium 3b, and the reference gas sensor 31 is provided above the culture medium 3b. The reference gas sensor 31 is provided at the same height as that of the gas sensor 21 by a string (not shown), for example. The reference gas sensor 31 has the same gas detection function as the gas sensor 21. As an example, the reference gas sensor 31 is a VOC sensor similar to the gas sensor 21.

The reference soil gas sensor 32 is disposed below the ground surface 3t of the second agricultural land 3n. In the example shown in FIG. 2, the reference soil gas sensor 32 is provided in the culture medium 3b. The reference soil gas sensor 32 is a VOC sensor that has the same gas detection function as the soil gas sensor 22. The reference soil gas sensor 32 detects gas or the like generated from the soil 3d of second agricultural land 3n.

The reference environment sensor 33 is disposed in the soil 3d of second agricultural land 3n. The reference environment sensor 33 has the same detection function as the environmental sensor 23. That is, when the environmental sensor 23 includes a moisture sensor, the reference environment sensor 33 also includes a moisture sensor that detects the moisture of the soil 3d. Also, when the environmental sensor 23 includes EC sensor, the reference environment sensor 33 also includes EC sensor which detects the soil 3d electrical conductivity. The position of the reference environment sensor 33 is not particularly limited as long as at least one of the moisture and electrical conductivity of the soil 3d can be detected.

The reference motion sensor 34 is disposed in the second agricultural land 3n. The reference motion sensor 34 detects people present in the second agricultural land 3n. The reference motion sensor 34 may be, for example, an infrared sensor disposed in the second agricultural land 3n. In addition, the reference motion sensor 34 may be constituted by an imaging device disposed in the second agricultural land 3n and a computer that analyzes an image captured by the imaging device, for example. The reference motion sensor 34 is not particularly limited as long as it is configured to be able to detect a person present in second agricultural land 3n. The reference motion sensor 34 is fixed by a string (not shown), for example.

Each detection data (an example of a detection result) of the reference gas sensor 31, the reference soil gas sensor 32, the reference environment sensor 33, and the reference motion sensor 34 is output to the control device 4.

The control device 4 has a controller 41 and a storage device 42. The controller 41 generally controls the monitoring system 1. The controller 41 is configured as, for example, a programmable logic controller (PLC). The controller 41 may be configured as a computer system including a processor such as a central processing unit (CPU), memories such as a random-access memory (RAM) and a read only memory (ROM), input/output devices such as a touch panel, a mouse, a keyboard, and a display, and a communication device such as a network card. The control device 4 realizes a function of outputting information about the plant T by operating each hardware under the control of the processor based on the computer program stored in the memory. In detail, the controller 41 outputs information about gas generated from the plant T and a state of the plant T based on the detection data acquired from the sensor group 2 and the detection data acquired from the reference sensor group 3. Details of specific processing by the controller 41 will be described later. The controller 41 outputs information about the plant T to the storage device 42.

The storage device 42 receives the information about the plant T from the controller 41, and stores the information related to the plant T. The storage device 42 is constituted by, for example, a hard disk drive (HDD). The storage device 42 may store a result acquired from an external input means or the like.

### [Monitoring Method]

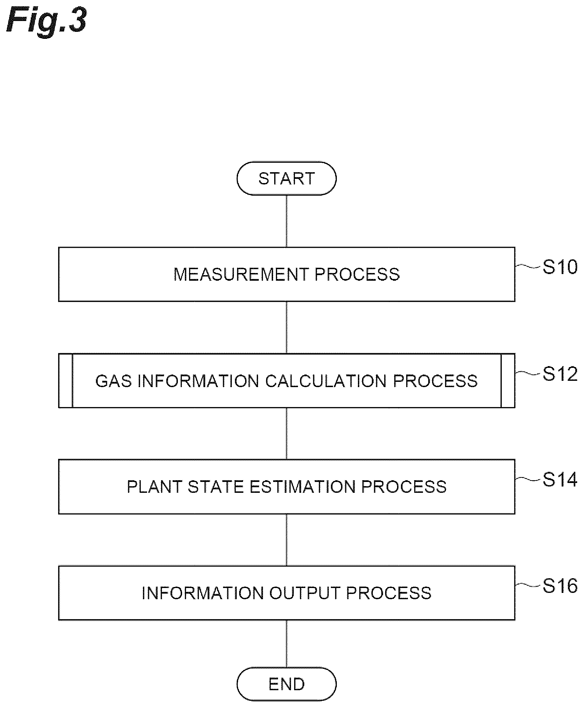

FIG. 3 is a flow chart showing the operation of the monitoring system 1. The monitoring method shown in FIG. 3 is performed by the monitoring system 1. The flowchart is started, for example, every time a predetermined time elapses.

First, as a measurement process (step S10), the sensor group 2 detects each value related to the first agricultural land 2n, and the reference sensor group 3 detects each value related to the second agricultural land 3n. First, measurement processing in the sensor group 2 will be described. The gas sensor 21 of the sensor group 2 detects gas generated from the plant T leaves, stems, or the like. Here, gas or the like generated from the soil 2d may be present near the gas sensor 21. Thus, in addition to gas generated from the plant T, the gas sensor 21 may also detect gas generated from the soil 2d, or the like. The gas sensor 21 outputs the detection data (an example of a first detection result) to the controller 41. The detection data detected by the gas sensor 21 includes at least the type of gas component detected. The detection data may further include a concentration of the detected gas component.

The soil gas sensor 22 detects gas generated from the root Tr of the plant T. Here, gas or the like generated from the soil 2d in the first agricultural land 2n may present near the soil gas sensor 22. Thus, in addition to gas generated from the root Tr of the plant T, the soil gas sensor 22 may also detect gas generated from the soil 2d of first agricultural land 2n. The soil gas sensor 22 outputs the detection data (an example of a third detection result) to the controller 41. The detection data detected by the soil gas sensor 22 includes at least the type of gas component detected. The detection data may further include a concentration of the detected gas component.

The environmental sensor 23 detects at least one of moisture and electrical conductivity of the soil 2d of the first agricultural land 2n. The motion sensor 24 detects the person existing in the first agricultural land 2n when a person exists in the first agricultural land 2n. Each of the environmental sensor 23 and the motion sensor 24 outputs detection data to the controller 41.

Next, a measurement process in the reference sensor group 3 will be described. The reference gas sensor 31 of the reference sensor group detects gas present in the vicinity of the reference gas sensor 31. To be specific, the reference gas sensor 31 detects gas or the like generated from the soil 3d. The reference gas sensor 31 outputs the detection data (an example of a second detection result) to the controller 41.

The reference soil gas sensor 32 detects gas present near the reference soil gas sensor 32. To be specific, the reference soil gas sensor 32 detects gas or the like generated from the soil 3d of second agricultural land 3n. The reference soil gas sensor 32 outputs the detection data (an example of a fourth detection result) to the controller 41. The reference environment sensor 33 detects at least one of moisture and electrical conductivity of the soil 3d of the second agricultural land 3n. The reference motion sensor 34 detects the person existing in the second agricultural land 3n when a person exists in the second agricultural land 3n. Each of the reference environment sensor 33 and the reference motion sensor 34 outputs detection data to the controller 41.

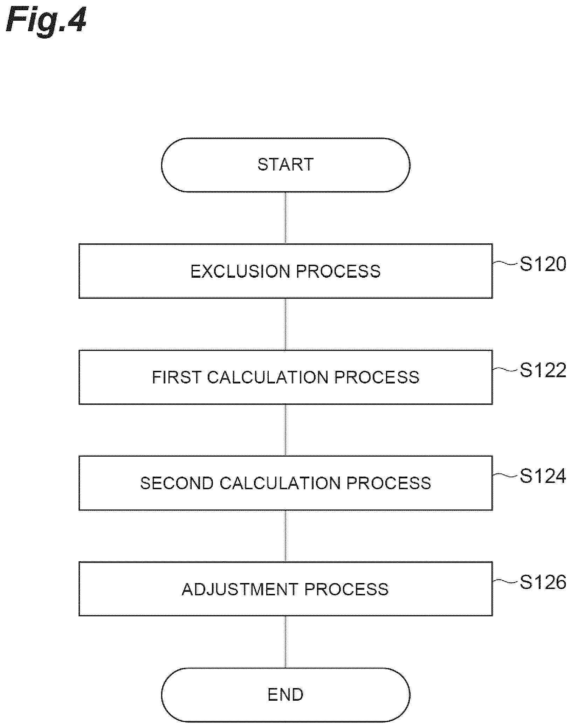

Subsequently, the controller 41 acquires each detection data detected by the sensor group 2 and each detection data detected by the reference sensor group 3, and executes a process illustrated in FIG. 4 as gas information calculation process (step S12). FIG. 4 is a flowchart showing details of the gas information calculation process. The controller 41 calculates information indicating gas generated from the plant T (an example of information related to gas generated from the plant T) by executing gas information calculation processing. The gas generated from the plant T includes gas generated from an upper portion of the plant T and gas generated from a lower portion of the plant T. Hereinafter, the gas information calculation process will be described in detail with reference to FIG. 4.

First, as an exclusion process (step S120), the controller 41 determines a noise data to be excluded from a detection data used in a first calculation process (step S122) to be described later among detection data in the gas sensor 21. To be more specific, the controller 41 refers to the detection data of the motion sensor 24, and determines, as the noise data, the detection data detected by the gas sensor 21 when a person in the first agricultural land 2n is detected by the motion sensor 24 among the detection data of the gas sensor 21. The controller 41 excludes the determined noise data from the detection data in the gas sensor 21 used in the first calculation process (step S122) described later. Thus, since the monitoring system 1 can exclude disturbance such as gas generated by a person passing near the plant T, the detection accuracy of gas generated from the plant T can be secured. At this time, the controller 41 may set the detection data detected by the reference gas sensor 31 at the same time as the noise data as the reference noise data, and exclude the reference noise data from the detection data in the reference gas sensor 31.

In addition, the controller 41 refers to the detection data of the reference motion sensor 34, and determines, as the reference noise data, the detection data detected by the reference gas sensor 31 when a person in the second agricultural land 3n is detected by the reference motion sensor 34 among the detection data of the reference gas sensor 31. Then, the controller 41 excludes the determined reference noise data from the detection data in the reference gas sensor 31 used in first calculation processing (step S122) described later. Thus, since the monitoring system 1 can exclude disturbance such as gas generated by a person passing through the second agricultural land 3n, detection accuracy of gas generated from the plant T can be secured. At this time, in the controller 41, a detection data detected by the gas sensor 21 at the same time as the reference noise data may be set as a noise data, and the noise data may be excluded from the detection data in the gas sensor 21.

Subsequently, as first calculation process (step S122), the controller 41 calculates information about gas generated from the plant T based on the detection data of the gas sensor 21 from which the noise data has been removed and the detection data of the reference gas sensor 31 from which the reference noise data has been removed. The first calculation process is a process of calculating the data of gas (hereinafter referred to as "plant upper gas") generated from a portion above the ground surface 2t of the first agricultural land 2n in the plant T such as leaves, stems, fruits or the like. The controller 41 compares the detection data of the gas sensor 21 with the detection data of the reference gas sensor 31. The first detection data of the gas sensor 21 used for comparison is data detected at the same time as the first detection data of the reference gas sensor 31 used for comparison.

As an example of a comparison method between the detection data of the gas sensor 21 and the detection data of the reference gas sensor 31, the following two methods are given. As a first method, when a data related to a common gas component between the detection data of the gas sensor 21 and the detection data of the reference gas sensor 31 is included, the controller 41 excludes the data related to the common gas component from the detection data of the gas sensor 21. Then, the controller 41 calculates the detection data of the gas sensor 21 excluding the data related to the common gas component as the data of the plant upper gas. For example, when the detection data of the gas sensor 21 includes the data of the first gas component and the second gas component, and the detection data of the reference gas sensor 31 includes the data of second gas component, the controller 41 excludes the data of the second gas component from the detection data of the gas sensor 21, and sets only the first gas component as the data of the plant upper gas. Thus, the monitoring system 1 can reliably remove the disturbance other than the plant upper gas from the detection data of the gas sensor 21.

As a second method, in a case where a data related to a common gas component between detection data of the gas sensor 21 and detection data of the reference gas sensor 31 is included, the controller 41 calculates the data of the plant upper gas in consideration of the concentration of the common gas component indicated by each detection data. For example, when the detection data of the gas sensor 21 includes the data of the first gas component having a concentration of 50% and the detection data of the reference gas sensor 31 includes the data of the first gas component having a concentration of 20%, the controller 41 calculates the data of the first gas component having a concentration of 30%, which is obtained by subtracting the concentration of 20% from the concentration of 50%, as the data of the plant upper gas. In this case, even when the same gas as the gas generated from the plant T is generated from an object different from the plant T in the first agricultural land 2n, the monitoring system 1 can appropriately output the data of the plant upper gas. The controller 41 may perform both the first method and the second method, or may perform one of the first method and the second method.

Subsequently, as second calculation process (step S124), the controller 41 calculates information related to gas generated from the plant T based on the detection data in the soil gas sensor 22 and the detection data in the reference soil gas sensor 32. The second calculation process is a process of calculating the data of gas (hereinafter referred to as "plant lower gas") generated from a portion lower than the ground surface 2t of the first agricultural land 2n in the plant T such as the root Tr of the plant T. For example, the controller 41 compares the detection data of the soil gas sensor 22 with the detection data of the reference soil gas sensor 32. The detection data of the soil gas sensor 22 used for comparison is the data detected at the same time as the detection data of the reference soil gas sensor 32 used for comparison. For example, the controller 41 performs at least one of the first method and the second method as a comparison method.

Subsequently, as adjustment process (step S126), the controller 41 adjusts the information indicating the plant upper gas calculated by the step S122 based on the detection data of the environmental sensor 23 and the detection data of the reference environment sensor 33. As an example, when the detection data of each of the environmental sensor 23 and the reference environment sensor 33 includes the detection data of moisture sensor, the controller 41 calculates a difference between the detection data of the environmental sensor 23 and the detection data of the reference environment sensor 33. The storage device 42 stores in advance, for example, the difference of the detection data of the moisture sensor and the correction coefficient of the detection data of the gas sensor 21 in association with each other. The correction coefficient is a coefficient for adjusting the gas discharge amount generated according to the difference of the moisture amount. The controller 41 adjusts the detection data in the gas sensor 21 by applying a correction factor to the detection data in the gas sensor 21. The storage device 42 may store the difference of the detection data of the moisture sensor and the correction coefficient of the detection data of the soil gas sensor 22 in association with each other in advance. The controller 41 may adjust the detection data in the soil gas sensor 22 by applying a correction factor to the detection data in the soil gas sensor 22.

In addition, when the detection data of each of the environmental sensor 23 and the reference environment sensor 33 includes the detection data in EC sensor, the controller 41 calculates a difference between the detection data of the environmental sensor 23 and the detection data of the reference environment sensor 33. In the storage device 42, for example, a difference of detection data in EC sensor and a correction coefficient of detection data in the gas sensor 21 are stored in association with each other in advance. The correction coefficient is a coefficient for adjusting a gas discharge amount generated according to a difference in electrical conductivity. The controller 41 adjusts the detection data in the gas sensor 21 by applying a correction factor to the detection data in the gas sensor 21. The storage device 42 may store the difference of the detection data in EC sensor and the correction coefficient of the detection data in the soil gas sensor 22 in association with each other in advance. The controller 41 may adjust the detection data in the soil gas sensor 22 by applying a correction factor to the detection data in the soil gas sensor 22. Accordingly, since the monitoring system 1 can grasp the environmental difference between the first agricultural land 2n and the second agricultural land 3n and adjust the influence of the difference on the gas detection data, the gas generated from the plant T can be more accurately detected.

Returning to the processing of FIG. 3, as plant state estimation process (step S14), the controller 41 estimates the state of the plant T based on information indicating gas generated from the plant T. For example, the storage device 42 stores a table in which a gas component generated from the plant T is associated with the state of the plant T. The controller 41 estimates the state of the upper part of the plant T with reference to the storage device 42. Thus, the monitoring system 1 can accurately estimate the state of the plant T on the basis of the gas generated from the plant T and accurately detected.

For example, when the gas component indicated by the plant upper part information (plant upper gas) is the green leaf volatile, the controller 41 can estimate the eating damage state of the plant T. When the gas component indicated by the plant upper part information is the terpene, the controller 41 can estimate the environmental stress state of the plant T. In addition, when the gas component indicated by the plant upper part information is the sulfur-based substance, the controller 41 may estimate a base state of the plant T. Further, when the gas component indicated by the plant upper part information is the moldy odor substance, the controller 41 can estimate the mold occurrence of the plant T. In addition, the controller 41 may estimate the state of the lower portion of the plant T by a method similar to the case of estimating the state of the upper portion of the plant T with reference to the gas component indicated by the plant lower portion information.

Subsequently, as information output process (step S16), the controller 41 outputs the plant upper part information and the plant lower part information calculated by the step S12, and the state of the plant T estimated in the step S14, and stores them in the storage device 42. When the information output process ends, the flowchart shown in FIG. 3 ends. The first calculation process (step S122) may be performed after the second calculation process (step S124), or may be performed in parallel with the second calculation process. Further, the exclusion process (step S120) may be performed after the first calculation process (step S122) and the second calculation process.

### [Summary of Embodiment]

The controller 41 outputs information about gas generated from the plant T based on the detection data of the gas sensor 21 and the detection data of the reference gas sensor 31. That is, in this the monitoring system 1, not only the gas sensor 21 is provided in an agricultural land where the plant T is cultivated but also the reference gas sensor 31 is provided in a comparative environment with respect to an agricultural land where the plant T is not cultivated, and information related to gas generated from the plant T is output based on each detection data. To be specific, the detection data of the gas sensor 21 disposed in an agricultural land where the plant T is cultivated is compared with the detection data of the reference gas sensor 31 disposed in an agricultural land where the plant T is not cultivated and having the same gas detection function as the gas sensor 21. As a result, even if the detection data of the gas sensor 21 includes components other than gas generated from the plant T, the monitoring system 1 can exclude disturbance caused by other than the plant T from the detection data of the gas sensor 21 on the basis of the detection data of the reference gas sensor 31. Therefore, according to this the monitoring system, gas generated from the plant T can be measured with high accuracy.

Plants release gas (odor) by being damaged by eating or being infected with viruses. In recent years, gas generated from a plant placed in a closed space such as a laboratory has been analyzed. However, in an actual agricultural land where a plant is cultivated, gas generated from other than the plant, such as gas generated from soil or the like, may exist. Therefore, there is a problem that gas other than gas generated from a plant is included in gas detected by a gas sensor disposed in the vicinity of the plant even when gas generated from the plant in an actual agricultural land is to be detected. The monitoring system 1 can clearly sense gas generated from a plant because the reference gas sensor 31 is also disposed in an agricultural land where the plant T is not cultivated and a gas component detected by the reference gas sensor 31 is excluded as disturbance from a data where the gas sensor 21 is detected.

In the monitoring system 1, the gas sensor 21 is provided above the ground surface 2t of the first agricultural land 2n, and the reference gas sensor 31 is provided above the ground surface 3t of the second agricultural land 3n. Thus, gas generated from stems, leaves, or the like of the plant T being present above the ground surface 2t can be accurately measured.

The monitoring system 1 includes the soil gas sensor 22 disposed on the lower side of the ground surface 2t of the first agricultural land 2n, and the reference soil gas sensor 32 disposed on the lower side of the ground surface 3t of the second agricultural land 3n. As a result, the monitoring system 1 can accurately measure gas generated from the root Tr or the like of the plant T existing on the lower side of the ground surface 2t in addition to gas generated from the stem and leaf of the plant T existing on the upper side of the ground surface 2t of the first agricultural land 2n.

### [Modification]

While various exemplary embodiments have been described above, various omissions, substitutions and changes may be made without being limited to the exemplary embodiments described above.

The controller 41 may not output information (specifically, plant lower part information) indicating the state of the plant T based on the detection data of the soil gas sensor 22 and the detection data of the reference soil gas sensor 32. In that case, the monitoring system 1 may not include the soil gas sensor 22 and the reference soil gas sensor 32. The controller 41 may not output information indicating the state of the plant T based on the detection data of the environmental sensor 23 and the detection data of the reference environment sensor 33. In that case, the monitoring system 1 may not include the environmental sensor 23 and the reference environment sensor 33. The controller 41 may not be processed to exclude the detection data of the gas sensor 21 in consideration of the detection data of the motion sensor 24. In that case, the monitoring system 1 may not include the motion sensor 24. The controller 41 may not be processed to exclude the detection data of the reference gas sensor 31 in consideration of the detection data of the reference motion sensor 34. In that case, the monitoring system 1 may not include the reference motion sensor 34.

The monitoring system 1 may include only the gas sensor 21, the reference gas sensor 31, and the controller 41. In this case, the controller 41 may calculate gas (specifically, plant upper gas) generated from the plant T based on only the detection data of the gas sensor 21 and the detection data of the reference gas sensor 31, and estimate the state of the plant T based on the gas.

The gas sensor 21 of the present disclosure may be the soil gas sensor 22 and the reference gas sensor 31 of the present disclosure may be the reference soil gas sensor 32. This also allows the monitoring system 1 to accurately measure gas generated from the root Tr or the like of the plant T that is present below the ground surface 2t. In that case, the monitoring system 1 may include only the soil gas sensor 22, the reference soil gas sensor 32, and the controller 41. In this case, the controller 41 may calculate the data of gas (specifically, plant lower gas) generated from the plant T based on only the detection data of the soil gas sensor 22 and the detection data of the reference soil gas sensor 32, and estimate the state of the plant T based on the data of the gas.

The first agricultural land 2n may not include the culture medium 2b and the second agricultural land 3n may not include the culture medium 3b. In that case, the plant T may be cultivated on ground soil, such as fields of rice and other crops, orchards, pasturelands or the like.

## Claims

1. A monitoring system (1) comprising:
a first gas sensor (21) configured to be disposed in a first agricultural land (2n) for cultivating a plant (T) and configured to detect a gas component;
a second gas sensor (31) configured to be disposed in a second agricultural land (3n) in which the plant (T) is not cultivated and configured to have a same gas detection function as the first gas sensor (21); and
a controller (41) configured to output information about gas generated from the plant (T) based on a first detection result of the first gas sensor (21) and a second detection result of the second gas sensor (31),
**characterised in that** the controller (41) is configured to output information obtained by excluding a common gas component from the first detection result as the information about gas generated from the plant (T) when the common gas component is included between the first detection result and the second detection result.

2. The monitoring system (1) according to claim 1, wherein the first gas sensor (21) is configured to be provided above a ground surface (2t) of the first agricultural land (2n), and the second gas sensor (31) is configured to be provided above a ground surface (3t) of the second agricultural land (3n).

3. The monitoring system (1) according to claim 2, further comprising:
a third gas sensor (22) configured to be disposed below the ground surface (2t) of the first agricultural land (2n) and to detect a gas component; and
a fourth gas sensor (32) configured to be disposed below the ground surface (3t) of the second agricultural land (3n) and to have a same gas detection function as the third gas sensor (22),
wherein the controller (41) is configured to output the information about gas generated from the plant (T) further based on a third detection result of the third gas sensor (22) and a fourth detection result of the fourth gas sensor (32).

4. The monitoring system (1) according to claim 1, wherein the first gas sensor (21) is configured to be provided below a ground surface (2t) of the first agricultural land (2n), and the second gas sensor (31) is configured to be provided below a ground surface (3t) of the second agricultural land (3n).

5. The monitoring system (1) according to any one of claims 1 to 4, further comprising:
a first environment sensor (23) configured to be disposed in the first agricultural land (2n) and to detect at least one of moisture and electrical conductivity in the first agricultural land (2n); and
a second environment sensor (33) configured to be disposed in the second agricultural land (3n) and to have a same detection function as the first environment sensor (23),
wherein the controller (41) is configured to output the information about gas generated from the plant (T) further based on a detection result of the first environment sensor (23) and a detection result of the second environment sensor (33).

6. The monitoring system (1) according to any one of claims 1 to 5, wherein the controller (41) is configured to estimate a state of the plant (T) based on the information about gas generated from the plant (T).

7. The monitoring system (1) according to any one of claims 1 to 6, further comprising a first motion sensor (24) configured to detect a person present in the first agricultural land (2n), wherein the controller (41) is configured to exclude a first detection result detected when the person present in the first agricultural land (2n) is detected by the first motion sensor (24) from the first detection result used to output the information about gas generated from the plant (T).

8. The monitoring system (1) according to claim 7, further comprising a second motion sensor (34) configured to detect a person present in the second agricultural land (3n), wherein the controller (41) is configured to exclude a second detection result detected when the person present in the second agricultural land (3n) is detected by the second motion sensor (34) from the second detection result used to output the information about gas generated from the plant (T).

## Patentansprüche

1. Überwachungssystem (1), das Folgendes umfasst:
einen ersten Gassensor (21), der konfiguriert ist, um in einer ersten Agrarfläche (2n) zum Anbauen einer Pflanze (T) angeordnet zu werden und konfiguriert ist, um eine Gaskomponente zu erfassen;
einen zweiten Gassensor (31), der konfiguriert ist, um in einer zweiten Agrarfläche (3n), in der die Pflanze (T) nicht angebaut wird, angeordnet zu werden und konfiguriert ist, um eine gleiche Gaserfassungsfunktion wie der erste Gassensor (21) aufzuweisen; und
eine Steuereinheit (41), die konfiguriert ist, um Informationen über Gas, das von der Pflanze (T) erzeugt wird, auf Basis eines ersten Erfassungsergebnisses des ersten Gassensors (21) und eines zweiten Erfassungsergebnisses des zweiten Gassensors (31) auszugeben,
**dadurch gekennzeichnet, dass** die Steuereinheit (41) konfiguriert ist, um Informationen, die durch Ausschließen einer gemeinsamen Gaskomponente aus dem ersten Erfassungsergebnis erhalten werden, als die Informationen über Gas auszugeben, das von der Pflanze (T) erzeugt wird, wenn die gemeinsame Gaskomponente zwischen dem ersten Erfassungsergebnis und dem zweiten Erfassungsergebnis eingeschlossen ist.

2. Überwachungssystem (1) nach Anspruch 1, wobei der erste Gassensor (21) konfiguriert ist, um über einer Bodenfläche (2t) der ersten Agrarfläche (2n) bereitgestellt zu werden und der zweite Gassensor (31) konfiguriert ist, um über einer Bodenfläche (3t) der zweiten Agrarfläche (3n) bereitgestellt zu werden.

3. Überwachungssystem (1) nach Anspruch 2, weiter umfassend:
einen dritten Gassensor (22), der konfiguriert ist, um unter der Bodenfläche (2t) der ersten Agrarfläche (2n) angeordnet zu werden und um eine Gaskomponente zu erfassen; und
einen vierten Gassensor (32), der konfiguriert ist, um unter der Bodenfläche (3t) der zweiten Agrarfläche (3n) angeordnet zu werden und eine gleiche Gaserfassungsfunktion wie der dritte Gassensor (22) aufzuweisen,
wobei die Steuereinheit (41) konfiguriert ist, um die Informationen über Gas, das von der Pflanze (T) erzeugt wird, weiter auf Basis eines dritten Erfassungsergebnisses des dritten Gassensors (22) und eines vierten Erfassungsergebnisses des vierten Gassensors (32) auszugeben.

4. Überwachungssystem (1) nach Anspruch 1, wobei der erste Gassensor (21) konfiguriert ist, um unter einer Bodenfläche (2t) der ersten Agrarfläche (2n) bereitgestellt zu werden und der zweite Gassensor (31) konfiguriert ist, um unter einer Bodenfläche (3t) der zweiten Agrarfläche (3n) bereitgestellt zu werden.

5. Überwachungssystem (1) nach einem der Ansprüche 1 bis 4, weiter umfassend:
einen ersten Umweltsensor (23), der konfiguriert ist, um in der ersten Agrarfläche (2n) angeordnet zu werden und mindestens eines von Feuchtigkeit und elektrischer Leitfähigkeit in der ersten Agrarfläche (2n) zu erfassen; und
einen zweiten Umweltsensor (33), der konfiguriert ist, um in der zweiten Agrarfläche (3n) angeordnet zu werden und eine gleiche Erfassungsfunktion aufzuweisen wie der erste Umweltsensor (23),
wobei die Steuereinheit (41) konfiguriert ist, um die Informationen über Gas, das von der Pflanze (T) erzeugt wird, weiter auf Basis eines Erfassungsergebnisses des ersten Umweltsensors (23) und eines Erfassungsergebnisses des zweiten Umweltsensors (33) auszugeben.

6. Überwachungssystem (1) nach einem der Ansprüche 1 bis 5, wobei die Steuereinheit (41) konfiguriert ist, um einen Zustand der Pflanze (T) auf Basis der Informationen über Gas, das von der Pflanze (T) erzeugt wird, zu schätzen.

7. Überwachungssystem (1) nach einem der Ansprüche 1 bis 6, weiter umfassend einen ersten Bewegungssensor (24), der konfiguriert ist, um eine in der ersten Agrarfläche (2n) vorhandene Person zu erfassen, wobei die Steuereinheit (41) konfiguriert ist, um ein erstes Erfassungsergebnis, das erfasst wird, wenn die in der ersten Agrarfläche (2n) vorhandene Person von dem ersten Bewegungssensor (24) erfasst wird, aus dem ersten Erfassungsergebnis auszuschließen, das zum Ausgeben der Informationen über Gas verwendet wird, das von der Pflanze (T) erzeugt wird.

8. Überwachungssystem (1) nach Anspruch 7, weiter umfassend einen zweiten Bewegungssensor (34), der konfiguriert ist, um eine in der zweiten Agrarfläche (3n) vorhandene Person zu erfassen, wobei die Steuereinheit (41) konfiguriert ist, um ein zweites Erfassungsergebnis, das erfasst wird, wenn die in der zweiten Agrarfläche (3n) vorhandene Person von dem zweiten Bewegungssensor (34) erfasst wird, aus dem zweiten Erfassungsergebnis auszuschließen, das zum Ausgeben der Informationen über Gas verwendet wird, das von der Pflanze (T) erzeugt wird.

## Revendications

1. Système de surveillance (1) comprenant :
un premier capteur de gaz (21) configuré pour être disposé dans un premier terrain agricole (2n) pour cultiver une plante (T) et configuré pour détecter un composant gazeux ;
un deuxième capteur de gaz (31) configuré pour être disposé dans un second terrain agricole (3n) dans lequel la plante (T) n'est pas cultivée et configuré pour présenter une même fonction de détection de gaz que le premier capteur de gaz (21) ; et
un dispositif de commande (41) configuré pour produire en sortie des informations sur un gaz généré par la plante (T) sur la base d'un premier résultat de détection du premier capteur de gaz (21) et d'un deuxième résultat de détection du deuxième capteur de gaz (31),
**caractérisé en ce que** le dispositif de commande (41) est configuré pour produire en sortie des informations obtenues en excluant un composant gazeux commun du premier résultat de détection en tant que les informations sur un gaz généré par la plante (T) lorsque le composant gazeux commun est inclus entre le premier résultat de détection et le deuxième résultat de détection.

2. Système de surveillance (1) selon la revendication 1, dans lequel le premier capteur de gaz (21) est configuré pour être prévu au-dessus d'une surface de sol (2t) du premier terrain agricole (2n), et le deuxième capteur de gaz (31) est configuré pour être prévu au-dessus d'une surface de sol (3t) du second terrain agricole (3n).

3. Système de surveillance (1) selon la revendication 2, comprenant en outre :
un troisième capteur de gaz (22) configuré pour être disposé en dessous de la surface de sol (2t) du premier terrain agricole (2n) et pour détecter un composant gazeux ; et
un quatrième capteur de gaz (32) configuré pour être disposé en dessous de la surface de sol (3t) du second terrain agricole (3n) et pour présenter une même fonction de détection de gaz que le troisième capteur de gaz (22),
dans lequel le dispositif de commande (41) est configuré pour produire en sortie les informations sur un gaz généré par la plante (T) sur la base en outre d'un troisième résultat de détection du troisième capteur de gaz (22) et d'un quatrième résultat de détection du quatrième capteur de gaz (32).

4. Système de surveillance (1) selon la revendication 1, dans lequel le premier capteur de gaz (21) est configuré pour être prévu en dessous d'une surface de sol (2t) du premier terrain agricole (2n), et le deuxième capteur de gaz (31) est configuré pour être prévu en dessous d'une surface de sol (3t) du second terrain agricole (3n).

5. Système de surveillance (1) selon l'une quelconque des revendications 1 à 4, comprenant en outre :
un premier capteur d'environnement (23) configuré pour être disposé dans le premier terrain agricole (2n) et pour détecter au moins un paramètre parmi l'humidité et la conductivité électrique dans le premier terrain agricole (2n) ; et
un second capteur d'environnement (33) configuré pour être disposé dans le second terrain agricole (3n) et pour présenter une même fonction de détection que le premier capteur d'environnement (23),
dans lequel le dispositif de commande (41) est configuré pour produire en sortie les informations sur un gaz généré par la plante (T) sur la base en outre d'un résultat de détection du premier capteur d'environnement (23) et d'un résultat de détection du second capteur d'environnement (33).

6. Système de surveillance (1) selon l'une quelconque des revendications 1 à 5, dans lequel le dispositif de commande (41) est configuré pour estimer un état de la plante (T) sur la base des informations sur un gaz généré par la plante (T).

7. Système de surveillance (1) selon l'une quelconque des revendications 1 à 6,
comprenant en outre un premier capteur de mouvement (24) configuré pour détecter une personne présente dans le premier terrain agricole (2n), dans lequel
le dispositif de commande (41) est configuré pour exclure un premier résultat de détection détecté lorsque la personne présente dans le premier terrain agricole (2n) est détectée par le premier capteur de mouvement (24) à partir du premier résultat de détection utilisé pour produire en sortie les informations sur un gaz généré par la plante (T).

8. Système de surveillance (1) selon la revendication 7, comprenant en outre
un second capteur de mouvement (34) configuré pour détecter une personne présente dans le second terrain agricole (3n), dans lequel
le dispositif de commande (41) est configuré pour exclure un deuxième résultat de détection détecté lorsque la personne présente dans le second terrain agricole (3n) est détectée par le second capteur de mouvement (34) à partir du deuxième résultat de détection utilisé pour produire en sortie les informations sur un gaz généré par la plante (T).
